# FASCICULE DE BREVET EUROPEEN

(11) **EP 2 651 422 B1**
(45) Date de publication et mention de la délivrance du brevet: **18.10.2017**
(21) Numéro de dépôt: 11799669.4
(22) Date de dépôt: 14.12.2011
(51) Int. Cl.: A61K 35/644, A61K 35/02, A61K 33/06, A61K 8/26, A61Q 19/00, A61K 8/97, A23L 21/20, A23L 33/16, A61P 9/00, A61P 13/00, A61P 1/00, A61P 3/12, A61P 19/10, A61P 17/00

(54) **COMPOSITIONS A BASE D'ARGILE ET DE BEEPOLLEN, LEUR PROCEDE DE PREPARATION ET LEURS UTILISATIONS EN NUTRITION ET EN THERAPEUTIQUE**
ZUSAMMENSETZUNG BEINHALTEND TON UND BIENENPOLLEN, VERFAHREN ZUR HERSTELLUNG UND DEREN BENUTZUNG IN THERAPIE UND ERNÄHRUNG
COMPOSITIONS COMPRISING CLAY AND BEEPOLLEN, METHOD OF PREPARATION AND THERAPEUTICAL OR NUTRITIONAL USES THEREOF

(30) Priorité: 14.12.2010 FR 1060511
(43) Date de publication de la demande: 23.10.2013
(73) Titulaire: Laboratoire Beepratte, 75006 Paris (FR); Fregonese, Alexandra, 47310 Moncaut (FR)
(72) Inventeur: FREGONESE, Alexandra, F-47310 Moncaut (FR)
(74) Mandataire: Lavoix
(86) Numéro de dépôt international: PCT/EP2011/072756
(87) Numéro de publication internationale: WO 2012/080333

(56) Documents cités:
- EP-A1- 0 191 128
- WO-A1-2008/109182
- WO-A2-2007/075972
- US-A1- 2002 012 640
- US-A1- 2008 031 940
- DATABASE WPI Week 201041 Thomson Scientific, London, GB; AN 2010-G56311 XP002634573, & CN 101 711 799 A (BEIJING RESOURCE ANIMAL PHARM CO LTD) 26 mai 2010 (2010-05-26)
- DATABASE WPI Week 200676 Thomson Scientific, London, GB; AN 2006-731803 XP002634574, & CN 1 806 574 A (YU N) 26 juillet 2006 (2006-07-26)
- DATABASE WPI Week 200776, Derwent Publications Ltd., London, GB; AN 2007-806600 & CN 1 965 799 A (LIU J) 23 Mai 2007

## Description

Le pollen est l'élément fécondant mâle des fleurs et représente la seule source de protéines pour l'abeille. Il est recueilli par l'abeille de l'organe mâle de la fleur pour être transporté jusqu'à la ruche sous forme de petites pelotes. Il est alors souvent désigné par le terme « beepollen » pour distinguer ce pollen véhiculé par l'abeille de celui véhiculé par le vent. Ainsi récolté par l'abeille, le beepollen s'est enrichi en éléments probiotiques, tels que les ferments et levures, contenus dans la salive de l'abeille.

Du fait de sa constitution intrinsèque, le pollen est aussi un aliment très intéressant en nutrition humaine car le pollen frais est composé de protéines (± 25 %) comportant notamment les acides aminés indispensables à l'organisme, y compris ceux qu'il ne peut synthétiser (phénylalanine, tryptophane, tyrosine, lysine, thréonine, méthionine, cystéine, leucine, isoleucine, valine) et des enzymes et coenzymes (dont l'amylase, invertase, phosphatase, des transférases) ; des vitamines présentes en grand nombre : la totalité des vitamines du groupe B, la vitamine C, la vitamine D, la provitamine A, des vitamines K, E, rutine; oligo-éléments tels que le calcium, le magnésium, le phosphore, le fer, le cuivre, le manganèse ... ; minéraux (cuivre, fer, manganèse, phosphore silicium, soufre) ; stérols ; flavonoïdes ; pigments ; substances oestrogéniques ; facteurs de croissance ; sélénium ; glucides ; lipides, etc. En raison de ces apports nutritionnels importants, le pollen a une action métabolique étendue et confère de la vitalité pour une meilleure forme générale.

Par exemple, la richesse du pollen en phytooestrogènes tels que les isoflavones permet une protection contre les maladies cardiovasculaires, l'ostéoporose, le cancer prostatique. La présence de flavonoïdes dans le pollen laisse également suggérer un effet bénéfique sur la circulation.

Plus particulièrement, l'effet sur l'ostéoporose est également supporté par la stimulation osseuse chez le rat induite par la prise orale de pollen (Yamaguchi et al. J. of Health Science 2006, 43-49).

Plusieurs études ont également montré l'efficacité du pollen (Cernilton®, extrait normalisé de pollen de seigle récolté mécaniquement) dans le traitement de l'hyperplasie bénigne de la prostate ((Wilt et al. Cochrane Database Syst. Rev. 2000 (2) CD001042). L'activité de différents extraits de beepollen sur certains types de cancers de la prostate par apoptose cellulaire a également été rapportée (Wu et al. Phytother Res. 2007, 21(11), 1087-1091).

Eraslan et al. (Ecotoxicol. Environ. Saf 2009, 72(3), 931-937) ont décrit l'effet protecteur du beepollen lors de l'administration d'un hépatotoxique, laissant suggérer l'effet bénéfique du beepollen sur le foie et le métabolisme en général.
US2008/0031940 décrit des compositions thérapeutiques comprenant essentiellement de la quercétine, de la papaïne, du sel de calcium, du sel de zinc, ainsi que du beepollen et éventuellement des diluants tels que les kaolinites et les bentonites. L'amélioration des symptômes ménopausiques a été observée après administration d'une composition à base de pollen (Georgiev et al., Med. Gen. Med. 2004, 6(4), 46).

On a également reconnu que le pollen restaure l'équilibre nerveux, et améliore les états d'insomnie et de fatigue. Le pollen est également recherché pour la stimulation des fonctions gastriques (avec effets favorables sur l'appétit, la digestion et une régulation du transit digestif) et les troubles circulatoires. Ainsi, le pollen contribue à la régénération sanguine, notamment en augmentant le taux d'hémoglobine et il serait donc particulièrement indiqué chez les personnes anémiques ou les enfants ; il régule également la coagulation sanguine (antithrombine) et contribue à la protection cardio-vasculaire grâce à l'association d'agents de résistance et d'élasticité artério-capillo-veineux : la rutine, les vitamines C et E et la méthionine. La présence de vitamine A suggère également un effet bénéfique du pollen sur la prévention ou le traitement des désordres oculaires.

Outre ces bienfaits intrinsèques au pollen, le beepollen, s'il est délivré intact dans l'intestin, pourrait permettre l'administration de ferments lactiques contribuant à la flore intestinale essentielle à la santé.

Cependant, le pollen frais n'est pas stable. Ainsi, aujourd'hui, le pollen est essentiellement commercialisé sous deux formes.

Sous forme sèche qui facilite sa distribution (longue conservation, facilité d'utilisation), le pollen perd néanmoins l'essentiel des propriétés, notamment antibactériennes.

La forme congelée permet de maintenir les qualités du pollen mais en rend la commercialisation et la prise par le patient peu commodes, en raison de la chaîne du froid qu'il est nécessaire de maintenir.

Sec ou congelé, le pollen se présente sous forme de grains, présentant d'autres désavantages, tel que la difficulté de respecter une véritable posologie et un goût qui peut être jugé désagréable, tant pour les consommateurs finaux que pour la réalisation d'études cliniques.

Il est donc désirable de mettre à disposition une formulation permettant l'administration du beepollen sous une forme, de préférence naturelle, dans laquelle il a conservé les qualités du beepollen frais, notamment nutritionnelles et physiologiques précitées, tout en répondant aux exigences de stabilité et de facilité d'utilisation.

Les présents inventeurs ont désormais mis en évidence des synergies entre le beepollen et l'argile permettant de formuler de façon stable le beepollen et d'en conserver voire d'en optimiser les qualités nutritionnelles, physiologiques et thérapeutiques, de favoriser sa conservation, son administration, sa mise sur le marché, sa consommation et son assimilation et la réalisation d'études cliniques.

Ainsi, la présente invention concerne des combinaisons consistant en du beepollen et de l'argile. Lesdites combinaisons permettent de formuler le beepollen en répondant aux exigences précitées.

Du fait des propriétés d'absorption, rétention et adsorption de l'argile, la combinaison ainsi formée présente une structure intimement liée et pourra être également désignée ici de « complexe beepollen/argile ».

Les complexes beepollen/argile selon l'invention peuvent également être définis comme toute matière stable résultant de la fixation de beepollen provenant de toute espèce végétale sur une structure lamellaire silicatée.

Au sens de l'invention, on entend par « beepollen » le pollen prélevé après récolte par les abeilles. Il peut être prélevé par tout moyen notamment, juste avant son entrée dans la ruche (grâce à une trappe à pollen) ou dans la ruche selon les méthodes habituelles.

Ce terme « beepollen » désigne le pollen sous toute forme permettant le maintien de l'activité biologique du pollen frais. Il comprend donc le beepollen frais ainsi que le beepollen préalablement congelé notamment.

Dans le cadre de la présente invention, le beepollen peut être originaire de diverses espèces et variétés végétales et n'est pas limité à une fleur particulière. On peut ainsi citer le beepollen de seigle, de maïs, de tournesol, de ciste, de châtaigner, de kiwi, de saule, de coquelicot, de lavande, de bruyère ...

Le beepollen, notamment le beepollen congelé, peut être disponible commercialement par exemple chez Pollenergie.

Le beepollen peut également être de composition variable, notamment en fonction de la nature des plantes d'origine ainsi que de la période de la récolte ou encore du mode de récolte.

Ainsi, le beepollen frais convenant à l'invention contient généralement de 5% à 36% d'eau et de 64% à 95% de matières sèches, parmi celles-ci :
- 2,5% à 3,8% de sels minéraux ;
- 4,2% à 19,8% de lipides ;
- 8% à 30% d'albumine ;
- 5% à 7% d'amidon ;
- des vitamines ;
- des facteurs de croissance ;
- de l'acide folique ;
- des polyphénols ;
- des phytostérols;
- des ferments et levures.

On entend par « argile » un matériau comprenant des minéraux argileux tels que les silicates hydratés, notamment des silicates d'aluminium ou de magnésium. On préfère notamment les phyllosilicates présentant une structure feuilletée constituée d'un ou plusieurs feuillets.

Selon la classification de Jozja, basée sur l'épaisseur et la structure du feuillet, on distingue quatre groupes d'argiles:
i) Minéraux à 7 Å : Son épaisseur est d'environ 7 Å, c'est la catégorie des kaolinites (kaolin, halloysite ...)
ii) Minéraux à 10 Å : Son épaisseur est d'environ 10 Å, c'est la catégorie des illites (illite, glauconite, ..)
iii) Minéraux à 14 Å : Son épaisseur est d'environ 14 A, c'est la catégorie des smectites (montmorillonite, bentonite, ghassoul ...)
iv) Minéraux fibreux : L'épaisseur du feuillet est variable on les appelle chlorites ou minéraux fibreux (sepiolite, attapulgite ...)

Le terme « argile » selon l'invention fait donc référence à ces quatre types d'argile. A titre de minéraux argileux, on peut notamment citer l'illite, la chlorite, la glauconite, la kaolinite, l'attapulgite, la sépiolite et la montmorillonite. On préfère notamment, à titre d'argile, le kaolin, l'illite, la montmorillonite, l'attapulgite, et plus particulièrement la montmorillonite.

Les matériaux argileux présentent des caractéristiques physico-chimiques spécifiques telles qu'une charge électrique négative, qui doit être compensée par des cations situés dans les espaces interfoliaires. La capacité d'échanges cationiques (CEC) est spécifique du type de matériaux argileux. La CEC est définie comme le nombre de cations monovalents ou équivalents qu'il est possible de substituer aux cations compensateurs pour compenser la charge négative de 100 g d'argile. Elle est déterminée par une introduction progressive d'un réactif coloré (cations) dans une suspension aqueuse jusqu'à saturation. La CEC s'exprime généralement en mEq/100 g.

La structure en feuillets conduit également à la propriété spécifique des matériaux argileux à gonfler en présence d'eau. Le gonflement correspond à la pénétration de molécules d'eau dans l'espace interfoliaire et peut être de type cristallin (rupture des forces électrostatiques entre les feuillets chargés et les cations en présence d'eau) ou osmotique (formation d'un gel puis d'une suspension en présence d'eau plus importante).

Ainsi, les différents types d'argile précités présentent des CEC et des capacités d'absorption spécifiques. Ainsi, la montmorillonite présente une CEC comprise entre 80 et 150 mEq/100 g et une capacité d'absorption (propriété mesurée par la différence de poids entre l'argile sèche et l'argile humide) de l'ordre de 20 %.

Les complexes selon l'invention consistent généralement :
- de 4% à 90% de beepollen, et
- de 10% à 96% d'argile.

Les pourcentages s'entendant en poids.

Plus préférentiellement, les complexes selon l'invention consistent :
- de 20% à 40% de beepollen et
- de 60% à 80% d'argile,
et plus particulièrement :
- de 30% à 35% de beepollen et
- de 65% à 70% d'argile.

Les complexes selon l'invention permettent de maintenir le beepollen sous une forme dans laquelle il conserve son activité biologique. Les complexes selon l'invention permettent donc de maintenir une humidité suffisante pour conserver les ferments et levures, tout en permettant, et ce de façon inattendue, la stabilité du beepollen ainsi formulé.

Les complexes selon l'invention présentent avantageusement une granulométrie inférieure à 500 µm, un taux d'humidité inférieur à 15% et/ou une activité d'eau (aw) inférieure à 0,75.

Selon un autre objet, la présente invention concerne également le procédé de préparation des complexes selon l'invention. Ainsi, ledit procédé comprend l'étape de mélange de beepollen et d'argile.

Le mélange est avantageusement réalisé sous agitation en air ambiant. Généralement, les complexes sont préparés en veillant à ce qu'aucune contamination d'eau (sous forme liquide) ne soit possible lors de la fabrication du mélange (matériel bien sec).

La granulométrie désirée est obtenue au moyen d'un tamis de maillage de 500 µm.

Selon la présente invention, les complexes présentent une activité biologique propre liée au beepollen utilisé, et potentialisée par l'argile ; inversement, l'argile présente des propriétés intrinsèques telles que l'action protectrice de la muqueuse intestinale, ses pouvoirs anti-acide et anti-alcalin, sa capacité à fixer et à évacuer les gaz et toxines.

On entend ici par « activité biologique » l'effet nutritionnel ou physiologique, tel que thérapeutique, exercé par lesdits complexes chez les sujets qui les ont consommés.

Le beepollen est en effet connu pour ses propriétés nutritionnelles comme source concentrée de nutriments et ses propriétés physiologiques sur l'ostéoporose, les maladies cardio-vasculaires, le foie et le métabolisme, les troubles circulatoires, les symptômes ménopausiques, le système nerveux, le système gastro-intestinal, les désordres articulaires, oculaires, urinaires, la fatigue, les désordres de la prostate tel que l'hypertrophie bénigne de la prostate, la prostatite chronique, la prostatodynie et le cancer de la prostate, etc. Il est également utile pour ses propriétés en nutrition, dermatologie et cosmétodermatologie, y compris les phanères (ongles, cheveux), asthénie et fatigabilité, convalescence et post-chirurgie, déséquilibres hydro-électrolytiques, améliorations des conditions physiques.

Ainsi, les inventeurs ont mis en évidence des synergies mises en oeuvre par les complexes beepollen/argile, et notamment la capacité et la vitesse d'absorption du complexe, sa capacité de rétention, sa capacité d'adsorption, sa charge en ferments lactiques et son effet sur les microorganismes, généralement supérieurs à la somme des effets de chacun de ses constituants.

La potentialisation de ces propriétés permet d'envisager l'amélioration des effets du beepollen et/ou de l'argile ainsi administré(s) au sein d'un complexe selon l'invention.

Les complexes selon l'invention sont donc utiles à titre thérapeutique ou à titre nutritionnel, pour usage humain et/ou animal, et à titre préventif et/ou thérapeutique.

La présente invention concerne donc également une composition pharmaceutique comprenant un complexe selon l'invention.

Ladite composition peut ainsi constituer notamment une spécialité pharmaceutique au sens de la Directive Européenne 2001/83/CE.

Ladite composition peut également comprendre un ou plusieurs excipient(s) pharmaceutiquement acceptable(s).

Les compositions pharmaceutiques selon l'invention peuvent être présentées sous des formes destinées à l'administration par voie orale, sublinguale, topique, locale, intratrachéale, intranasale ou rectale.

Elles peuvent donc être notamment présentes sous forme de solutés ou flacons multi-doses, ou sous forme de comprimés nus ou enrobés, de dragées, de capsules, de gélules molles ou dures, de granules, pilules, de cachets, de poudres, de suppositoires ou de capsules rectales, de solutions ou de suspensions, ou encore de crèmes, gels, pommades, patch, etc.

Les excipients pharmaceutiquement acceptables qui conviennent pour de telles administrations peuvent être notamment choisis parmi les dérivés de la cellulose ou de la cellulose microcristalline, les carbonates alcalino-terreux, le phosphate de magnésium, les amidons, les amidons modifiés, le lactose pour les formes solides, le beurre de cacao ou les stéarates de polyéthylèneglycol, l'eau, les solutés aqueux, le sérum physiologique, les solutés isotoniques, etc.

La présente invention vise plus particulièrement les gélules comprenant un complexe beepollen/argile.

La formulation de telles gélules peut être réalisée selon la pratique habituelle.

De préférence, ladite composition contient une quantité efficace du complexe selon l'invention.

La posologie peut varier dans les limites importantes (0,5 mg à 1000 mg) en fonction de l'indication thérapeutique et de la voie d'administration, ainsi que de l'âge et du poids du sujet.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé selon le mode d'administration, le poids et la réponse dudit sujet.

La présente invention concerne également un complexe selon l'invention pour utilisation dans la prévention et/ou le traitement de l'ostéoporose, des maladies cardio-vasculaires, des cancers, des désordres du foie et du métabolisme, de la convalescence, des troubles circulatoires, des troubles articulaires, des symptômes ménopausiques, des désordres du système nerveux, des désordres du système gastro-intestinal, des désordres oculaires, urinaires, des désordres de la prostate tel que l'hypertrophie bénigne de la prostate, la prostatite chronique, la prostatodynie et le cancer de la prostate, troubles nutritionnels, dermatologiques, asthénie, post-chirurgie et déséquilibres hydro-électrolytiques.

Il est également décrit un complexe selon l'invention pour utilisation dans l'amélioration de conditions physiques, le traitement et/ou la prévention de la fatigabilité, des désordres cosmétodermatologiques.

L'invention concerne également l'utilisation non thérapeutique d'une composition selon l'invention pour le traitement de la fatigue.

Selon un autre objet, la présente invention concerne également l'utilisation à titre nutritionnel d'un complexe selon l'invention.

On entend par « utilisation à titre nutritionnel », l'utilisation d'un complexe selon l'invention comme ou dans un aliment fonctionnel (ou alicament) ou un complément alimentaire.

Les aliments fonctionnels ou alicaments sont généralement définis comme un aliment conventionnel, ou qui en a l'apparence, qui fait partie de l'alimentation normale, et qui a pour caractéristique de procurer des effets physiologiques bénéfiques dépassant ses fonctions nutritionnelles habituelles ou de réduire le risque de maladies chroniques.

Ledit complément peut ainsi constituer un complément alimentaire au sens de la Directive Européenne 2002/46/CE.

En effet, les complexes selon l'invention constituent une denrée alimentaire dont le but est de compléter le régime alimentaire normal en constituant une source concentrée de nutriments (notamment les vitamines B, provitamine A et vitamines C, D et E, et minéraux précités) et les autres substances précitées ayant un effet nutritionnel et/ou physiologique en combinaison.

La présente invention concerne donc une composition alimentaire, telle que un aliment fonctionnel ou un complément alimentaire comprenant un complexe beepollen/argile selon l'invention.

Ledit complément alimentaire est avantageusement formulé sous forme de dose, à savoir dans une forme de présentation définie par la Directive Européenne 2002/46/CE, telle que « *les gélules, les pastilles, les comprimés, les pilules et autres formes similaires, ainsi que les sachets de poudre, les ampoules de liquide, les flacons munis d'un compte-goutte et les autres formes analogues de préparations liquides ou en poudre destinées à être prises en unités de faible quantité ».*

Le complément alimentaire selon l'invention se présente avantageusement sous forme de gélules. Il peut également comprendre des excipients alimentaires classiques.

La présente invention concerne également les combinaisons comprenant un complexe beepollen/argile selon l'invention avec un autre agent actif.

Les complexes beepollen/argile selon l'invention peuvent en effet être utiles pour potentialiser l'activité commune dudit agent et du complexe, ou à titre de véhicule pour en améliorer la formulation, l'administration et/ou la biodisponibilité, ou encore pour en diminuer les effets secondaires.

Ainsi, la présente invention concerne également un médicament comprenant à titre de véhicule une combinaison d'argile et de beepollen selon l'invention.

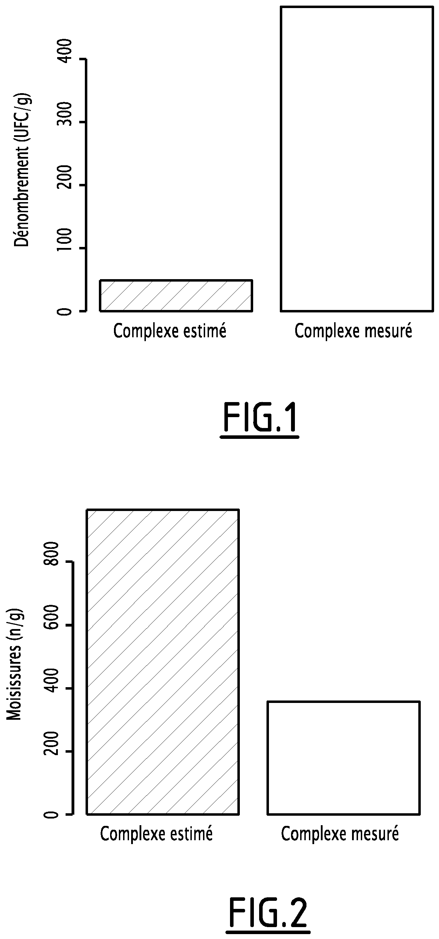

Les figures 1 à 6 représentent respectivement la charge en bactéries lactiques, en moisissures, et la capacité d'absorption, vitesse d'absorption, pouvoir de rétention et capacité d'adsorption d'un complexe beepollen/argile selon l'invention comparativement à une valeur estimée sur la base des propriétés des chacun de ses ingrédients.

Les exemples suivants illustrent l'invention, sans toutefois la limiter. Les produits de départs utilisés sont des produits connus ou préparés selon des modes opératoires connus.

Les pourcentages sont exprimés en poids, sauf mention contraire.

### Exemple 1 : Préparation de complexes beepollen/argile

### Matériel:

Balance électronique de précision- Mettler Toledo PG803
Agitateur - IKA Labotechnik Eurostar
Bêcher en verre 250 mL - Bomex
Spatules courbées, forme cuillère, acier inox 18/8- Labo-moderne

### Fournisseurs de matières premières:

Beepollen de Tournesol congelé sous gaz azote : Pollenergie, La Grabère - 47450 Saint-Hilaire de Lusignan ; Tél. 05 53 68 11 11 - Fax. 05 53 68 11 12

Argile Montmorillonite, Argiletz, 14 route d'Echampeau - 77 440 Liz-sur-Ourcq, Tél. 01 60 61 20 88 - Fax. 01 60 61 27 39.

9 compositions différentes (%) du complexe ont été réalisées :
- 100 argile / 0 beepollen,
- 90 argile / 10 beepollen,
- 80 argile /20 beepollen,
- 66,66 argile /33,33 beepollen,
- 50 argile / 50 beepollen,
- 33,33 argile /66,66 beepollen,
- 20 argile /80 beepollen,
- 10 argile / 90 beepollen,
- 0 argile / 100 beepollen.

Le mode opératoire illustratif est décrit plus en détail ci-après :
Pour la préparation de 150 g de complexe beepollen/argile : les proportions de chacun des composants est de 66,66 % d'argile pour 33,33% de beepollen.
   - Peser 100 g d'argile dans un bécher de 250 mL ;
   - Peser 50 g de beepollen congelé dans un bécher de 250 mL ;
   - Les pièces de l'agitateur sont de préférence sèches pour éviter l'hydratation du complexe ;
   - Mélanger l'argile ;
   - Ajouter le beepollen et laisser mélanger pendant 30 secondes.

La granulométrie désirée est obtenue par tamisage au moyen d'un tamis de maillage 500µm.

Si on place un seuil à 10% de l'effet maximum, on détermine une fenêtre dans laquelle les complexes présentent un effet significatif correspondant à :
- beepollen : de 4,5% à 87,6% avec une préférence à 33,33% ;
- argile: de 95,5% à 12,4% avec une préférence à 66.66%.

Les complexes ainsi préparés ont été caractérisés. Leurs caractéristiques produits peuvent être résumés dans le tableau ci-après :

| **ANALYSES** | **SPECIFICATIONS** |
|---|---|
| Aspect | Poudre |
| Couleur | Gris - vert |
| Odeur | Spécifique |
| Goût | Amer |
| Texture | Granuleuse |
| Granulométrie | Inférieur à 500µm |
| Corps étrangers | Absence |
| Densité | NA |
| Ph | NA |
| Humidité | Inférieur à 15% |
| Aw | Inférieur à 0.75 |
| Viscosité | NA |
| Valeur énergétique | 50kcal/100g< < 400kcal/100g |
| Lipides | 1%< <5% |
| Glucides | 15%< <28% |
| Protides | 3%< <11% |

Des essais pour évaluer l'efficacité du complexe sur la synergie des composants ont été réalisés. La composition 66,66 % d'argile et de 33,33 % de beepollen présente une efficacité maximum.

### Exemple 2 : Etude des propriétés du complexe beepollen/argile de l'exemple 1

### Dénombrement des bactéries lactiques totales

Les espèces de bactéries lactiques contenues dans le beepollen ne sont pas clairement identifiées. Le test qui permettait de dénombrer la plus grande majorité des espèces de bactéries lactiques (norme ISO 15214) a donc été utilisé.
▪ *Bactéries lactiques des ingrédients (argile et beepollen) :* Pour évaluer les avantages du complexe beepollen/argile, la quantité arbitraire en bactéries lactiques de chacun des ingrédients a tout d'abord été estimée.

L'argile présente 0 UFC/g.

Le beepollen présente 152 UFC/g.
▪ *Bactéries lactiques du complexe beepollen*/*argile :* Grâce au dénombrement spécifique du beepollen et de l'argile, la quantité de bactéries lactiques du complexe (en tenant compte de la proportion d'argile et de beepollen du complexe) a donc été estimée (Figure 1).

Ainsi, le complexe beepollen/argile devrait, selon notre estimation, contenir 50,7 UFC/g.

Or, les mesures montrent que le complexe beepollen/argile contenait 481 UFC/g.

Le complexe présente 9,5 fois plus de bactéries lactiques que l'estimation.

### Développement des moisissures

Moisissure est un nom vernaculaire ambigu qui désigne en français certains microorganismes microscopiques filamenteux du règne des mycètes. Il en existe des milliers de variétés différentes. Ce sont des *organismes* pluricellulaires qui peuvent atteindre jusqu'à 35 mètres de longueur.

Certaines moisissures sont sources d'intoxication alimentaire par les mycotoxines qu'elles sécrètent (patuline, etc.).
▪ *Moisissures des ingrédients (argile et beepollen) :* Pour évaluer les avantages du complexe beepollen argile, la quantité de moisissures de chacun des ingrédients a tout d'abord été estimée.

L'argile présente 0 moisissure/g.

Le beepollen présente 2900 moisissures/g.
▪ *Moisissures du complexe beepollen-argile :* Grâce au dénombrement spécifique du beepollen et de l'argile, la quantité de moisissures du complexe (en tenant compte de la proportion d'argile et de beepollen du complexe) a été estimée (Figure 2).

Ainsi, le complexe beepollen-argile devrait, selon cette estimation, contenir 967 moisissures/g. Or, les mesures montrent que le complexe beepollen-argile contenait 350 moisissures/g.

### Pouvoir absorbant du complexe beepollen-argile

Le pouvoir absorbant a été mesuré selon le protocole suivant :
1) Placer un filtre sur un bécher contenant de l'eau. L'eau humidifie progressivement le filtre qui ne plonge que très légèrement dans l'eau.
2) Déposer délicatement l'argile (ou le beepollen ou le complexe) sur le papier filtre. La matière déposée directement en contact avec le papier filtre absorbe l'eau.
3) Par capillarité, l'absorption d'eau se réalise progressivement vers le centre de la matière déposée.
4) L'argile devient complètement imbibée d'eau. La différence de poids entre l'argile sèche et l'argile humide donne la capacité d'absorption de l'argile.

Dans un premier temps, la capacité d'absorption de chacun des ingrédients a été mesurée :
- l'argile a la capacité d'absorber 74,38 % de son poids ;
- le beepollen a la capacité d'absorber 19,46 % de son poids.

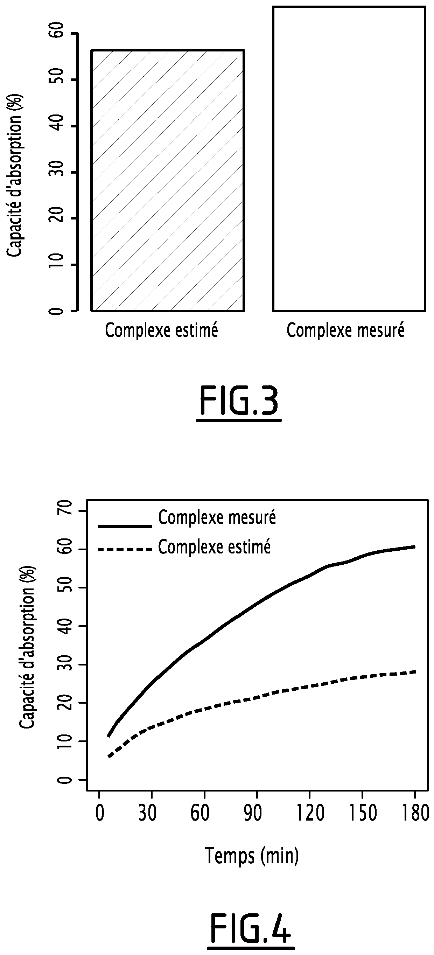

Grâce à la mesure de la capacité d'absorption spécifique du beepollen et de l'argile, la capacité d'absorption du complexe a été estimée en tenant compte de la proportion d'argile et de beepollen du complexe. Ainsi, le complexe beepollen-argile devrait, selon cette estimation, absorber 56% de son poids. Or, la mesure montre que le complexe beepollen-argile absorbe 65,5% de son poids. Le complexe beepollen-argile présente donc un pouvoir absorbant de 9,5 % supérieur par rapport à l'estimation (Figure 3).

La vitesse d'absorption a également été mesurée pour chacun des ingrédients à différents temps après la mise en contact avec de l'eau et en évaluant aux différents points la pente de la courbe. Ainsi, l'argile possède une vitesse d'absorption supérieure au beepollen. Grâce à la mesure de la vitesse d'absorption spécifique du beepollen et de l'argile, la capacité d'absorption du complexe aux différents temps mesurés a été estimée en tenant compte de la proportion d'argile et de beepollen du complexe. Les mesures réalisées avec le complexe montrent que le complexe beepollen/argile absorbe l'eau plus rapidement que l'estimation issue de la somme des deux ingrédients (en deux heures de temps, le pourcentage absorbé est 2,2 fois supérieur), et également plus rapidement que l'argile seule (en deux heures de temps, le pourcentage absorbé est 1,8 fois supérieur). Il y a donc un effet synergique du beepollen et de l'argile sur la capacité d'absorption (Figure 4).

### Capacité de rétention du complexe beepollen/argile

Pour évaluer la capacité de rétention, les produits (argile, beepollen et complexe) ont été laissés à absorber le maximum d'eau. Ainsi, le volume d'eau absorbée pour chaque produit (correspondant aux valeurs obtenues lors de la mesure d'absorption) a été déterminé, puis les produits ont été laissés se déshydrater naturellement (température ambiante et taux d'humidité contrôlés) et la perte d'eau a été calculée (pourcentage par rapport au volume absorbé. Les résultats montrent que :
- le beepollen se déshydrate plus rapidement que l'argile ;
- l'argile perd complètement (et seulement) l'eau qu'elle a absorbée alors que le beepollen perd non seulement l'eau absorbée mais également l'eau contenue dans le grain de beepollen.

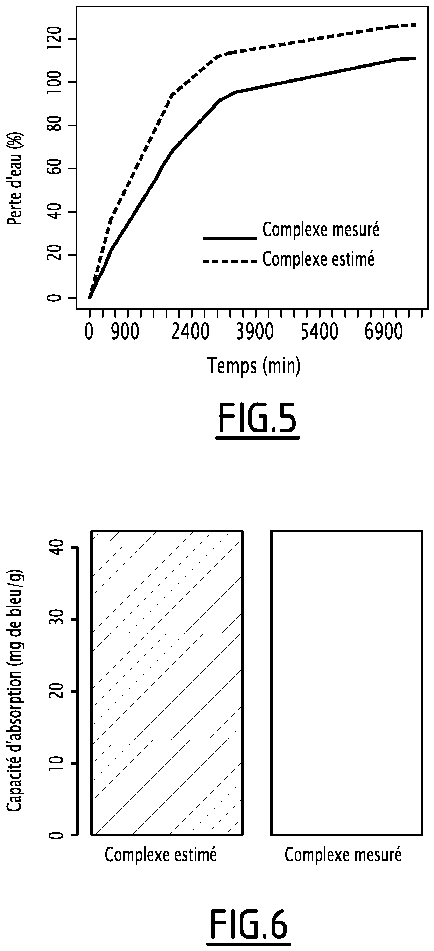

Après absorption maximale, la matière a été retirée du contact avec l'eau. La perte d'eau est ensuite mesurée à différents temps et représentée en pourcentage d'eau maximale absorbée. Grâce aux mesures des capacités de rétention d'eau pour les différents ingrédients, la capacité de rétention du complexe beepollen-argile a été estimée. Les mesures obtenues expérimentalement avec le complexe mettent en évidence un pouvoir de rétention plus important que celui estimé au regard des proportions et des capacités respectives de ses composants. Le complexe retient mieux les liquides que l'estimation (Figure 5).

Après plus de 5 jours à température ambiante (humidité contrôlée), le complexe retient 15% de son poids en plus que ce qui est attendu. La formulation de beepollen et d'argile permet d'obtenir un produit dont la capacité de rétention est potentialisée. Ces caractéristiques montrent la possibilité d'ajouter des produits qui seront conservés dans le produit final.

### Capacité d'adsorption

Le pouvoir adsorbant peut être déterminé par une introduction progressive d'un réactif coloré (cation) dans une suspension aqueuse d'argile jusqu'à saturation. Un essai au bleu de méthylène a été réalisé : c'est un cation coloré qui a été adsorbé de façon préférentielle par les argiles de type montmorillonite. C'est un test communément utilisé en géotechnie pour déterminer la propreté d'un sol et la proportion d'argile qu'il contient (NF P 94-068).

L'essai est effectué en ajoutant successivement des doses d'une solution de bleu de méthylène dans une suspension aqueuse d'argile et par vérification de l'adsorption de la solution colorée par un test à la tache sur papier filtre pour déceler la présence de colorant libre. Le bleu de méthylène a la propriété d'être rapidement adsorbé par l'argile. Tant que le bleu de méthylène est adsorbé, il ne colore pas l'eau de la solution. On le vérifie en déposant une goutte sur du papier filtre : le centre de la tache est bleu vif (argile ayant adsorbé le bleu) et l'auréole de la tache reste incolore. A partir d'une certaine dose de bleu de méthylène, l'auréole se colore aussi : c'est le signe que toute l'argile a épuisé sa capacité d'adsorption (saturation). La quantité de bleu consommée est donc une indication de la capacité d'adsorption du produit testé. La valeur de bleu s'exprime par la quantité de bleu en gramme consommé par gramme de produit.

3 g d'argile (Montmorillonite) sont homogénéisés dans 20 mL d'eau déminéralisée. Des ajouts successifs d'1 mL de solution de bleu de méthylène à 10g/L sont réalisés jusqu'à saturation. La saturation correspond au moment où une auréole bleu se forme sur le papier filtre ; le résultat est confirmé en répétant le test de la tache toutes les minutes pendant 5 minutes sans ajout de solution de bleu de méthylène.

On remarque que l'auréole bleue persiste légèrement à partir de l'ajout de 19 mL de bleu de méthylène. En prenant cette valeur, on peut déterminer la valeur de bleu de l'argile de 63,3 mg de bleu/g d'argile.

3 g de beepollen sont homogénéisés dans 20 mL d'eau déminéralisée. Comme le beepollen ne présente pas de propriétés adsorbantes, des ajouts successifs de 0,1 mL (puis 1 mL à partir de 1 mL) de solution de bleu de méthylène à 10g/L sont réalisés. La saturation correspond au moment où une auréole bleue se forme sur le papier filtre ; le résultat est confirmé en répétant le test de la tache toutes les minutes pendant 5 minutes sans ajout de solution de bleu de méthylène. L'auréole bleue persiste légèrement dès l'ajout de 0,1 mL de bleu de méthylène. Par conséquent, le beepollen ne possède aucune capacité adsorbante (0 mg de bleu/g de beepollen).

3 g de complexe beepollen-argile sont homogénéisés dans 20 mL d'eau déminéralisée. Des ajouts successifs d'1 mL de solution de bleu de méthylène à 10g/L sont réalisés jusqu'à saturation. La saturation correspond au moment où une auréole bleue se forme sur le papier filtre. Le résultat est confirmé en répétant le test de la tache toutes les minutes pendant 5 minutes. A 12 mL de bleu ajouté, l'auréole persiste légèrement même après 25 minutes. Il s'agit du volume de saturation. La valeur de bleu du complexe beepollen-argile est donc de 42,2 mg de bleu/g d'argile (Figure 6).

Ces résultats montrent que la capacité d'adsorption du complexe estimée est conforme au pouvoir adsorbant mesuré. Néanmoins, ce test montre également que l'auréole bleue visible initialement disparaît dans le temps. Ceci montre que des échanges se produisent entre le beepollen et l'argile.

### Bilan des synergies entre l'argile et le beepollen

Connaissant les propriétés intrinsèques du beepollen et de l'argile, les propriétés suivantes du complexe beepollen/argile ont été établies. Les résultats dépassent largement les estimations et confirment les synergies exercées par la combinaison du beepollen et de l'argile.

L'ensemble des avantages (/estimations) est représenté dans le tableau suivant :

| | |
|---|---|
| ABSORPTION | +9,5% |
| *Empêche la déshydratation du grain de beepollen* | |
| RETENTION | + 15% |
| *Peut retenir des substances* | |
| ADSORPTION | = |
| *Peut retenir des molécules actives* et *les ferments lactiques* | |
| CAPACITE D'ECHANGE | 200% |
| *Favorise les échanges beepollen - argile* | |
| FERMENTS LACTIQUES | x 9,5 |
| *Augmente la charge de bactéries lactiques* | |
| MOISISSURES | /3 |
| *Réduit la formation de moisissures* | |
| | Effets supérieurs/ estimations |

### Synergie du complexe beepollen/argile

Ces caractéristiques confirment les propriétés extrêmement intéressantes du complexe puisque les caractéristiques du beepollen sont conservées et certains apports comme celui des ferments lactiques sont potentialisés. Les capacités de rétention vont permettre également d'ajouter de nouvelles molécules/substances qui seront adsorbées par le complexe.

Les exemples 1 et 2 ont été répétés avec d'autres types d'argiles : Kaolinites, illites, minéraux fibreux.

Les résultats sont résumés dans le tableau suivant :

| | **Argile représentative** | **Absorption** | **Rétention** | **Adsorption** | **Capacité d'Echange** | **Ferments lactiques** | **Moisissures** |
|---|---|---|---|---|---|---|---|
| **Kaolinites** | Kaolin | +11% | +1% | = | 20% | X3 | /1.3 |
| **Illites** | Illite | +12% | +2% | = | 55% | X2.5 | /1.2 |
| **Smectites** | Montmorillonite | +9.5% | +15% | = | 200% | X9.5 | /3 |
| **Chlorites ou Minéraux fibreux** | Attapulgite | +17% | +5% | = | 240% | X3.5 | /1.5 |

### Exemple 3 : Préparation d'une gélule

Une capsule à enveloppe dure, ou gélule, comportant une enveloppe préfabriquée constituée de deux parties cylindriques ouvertes à une extrémité et dont le fond est hémisphérique a été préparée. Le complexe sous forme solide (poudre ou granulés) est introduit dans l'une des deux parties, puis la seconde est emboîtée sur la première. Les capsules Vcaps®, commercialisés par Pfizer, ont été utilisées à cette fin. La tunique de la gélule est en hypromellose, certifiée sans amidon, sans gluten et sans conservateurs.

La gélule comprend 500 mg du complexe beepollen/argile selon l'exemple 1 par gélule. De façon illustrative, une gélule peut comprendre :
- argile : supérieur à 50% en poids
- beepollen : 25% à 50% en poids
- hypromellose : 10% à 25 % en poids
- dioxyde de titane : 0,1% à 1 %
- complexe de cuivre et de chlorophylline : inférieur à 0,1% en poids.

Tous ces ingrédients sont conformes à l'utilisation dans le cadre d'un complément alimentaire.

### Exemple 4 : Caractérisation des gélules

- valeur énergétique : 129 kcal/100 g
- lipides : 1,97 %
- glucides : 21,4%
- protides : 6,42 %
- cendre : 62 %.

Les caractéristiques physiques ont également été déterminées.
- granulométrie : 500 µm ;
- corps étrangers : absence.

Les caractéristiques chimiques suivantes ont été obtenues :
- pesticides, métaux lourds, nitrites et nitrates : conformes à la réglementation européenne en vigueur
- pH : non applicable
- humidité: 7,13 %
- activité d'eau aw : 0,675 à 24,4°C
- viscosité : non applicable
- densité : non applicable.

## Revendications

1. Combinaison consistant en du beepollen et de l'argile.

2. Combinaison selon la revendication 1 telle que l'argile est choisie parmi les argiles de type kaolinites, smectites, illites et chlorites ou minéraux fibreux.

3. Combinaison selon la revendication 1 ou 2 telle que l'argile est la montmorillonite.

4. Combinaison selon l'une quelconque des revendications précédentes consistant en :
- de 4 % à 90 % de beepollen, et
- de 10 % à 96 % d'argile,
les pourcentages s'entendant en poids.

5. Combinaison selon l'une quelconque des revendications précédentes consistant en :
- de 20 % à 40 % de beepollen et
- de 60 % à 80 % d'argile.

6. Combinaison selon l'une quelconque des revendications précédentes telle qu'elle présente une granulométrie inférieure à 500 µm, un taux d'humidité inférieur à 15% et/ou une activité d'eau (aw) inférieure à 0,75.

7. Procédé de préparation d'une combinaison selon l'une quelconque des revendications précédentes comprenant l'étape de mélange de beepollen et d'argile.

8. Composition pharmaceutique comprenant une combinaison selon l'une quelconque des revendications 1 à 6 et éventuellement un ou plusieurs excipient(s) pharmaceutiquement acceptable(s).

9. Combinaison selon l'une quelconque des revendications 1 à 6 pour utilisation dans la prévention et/ou le traitement de l'ostéoporose, des maladies cardio-vasculaires, des cancers, des désordres du foie et du métabolisme, des troubles circulatoires, des symptômes ménopausiques, des désordres du système nerveux, des désordres du système gastro-intestinal, des désordres oculaires, la convalescence, la post-chirurgie des troubles articulaires, urinaires, des désordres de la prostate tel que l'hypertrophie bénigne de la prostate, la prostatite chronique, la prostatodynie et le cancer de la prostate, les troubles nutritionnels, dermatologiques, asthénie et déséquilibres hydro-électrolytiques.

10. Composition alimentaire comprenant une combinaison selon l'une quelconque des revendications 1 à 6.

11. Gélule comprenant une combinaison consistant en de l'argile et du beepollen selon l'une quelconque des revendications 1 à 5.

12. Médicament comprenant à titre de véhicule une combinaison consistant en de l'argile et du beepollen selon l'une quelconque des revendications 1 à 5.

13. Utilisation non-thérapeutique d'une composition selon l'une quelconque des revendications 1 à 5 pour le traitement de la fatigue.

## Patentansprüche

1. Kombination, bestehend aus Bienenpollen und Ton.

2. Kombination nach Anspruch 1, wobei der Ton ausgewählt ist aus den Tonen des Typs Kaolinite, Smektite, Illite und Chlorite oder Fasermineralien.

3. Kombination nach Anspruch 1 oder 2, wobei der Ton Montmorillonit ist.

4. Kombination nach einem der vorhergehenden Ansprüche, bestehend aus:
- 4 % bis 90 % Bienenpollen, und
- 10 % bis 96 % Ton,
wobei die prozentualen Anteile als Gewichtsprozente zu verstehen sind.

5. Kombination nach einem der vorhergehenden Ansprüche, bestehend aus:
- 20 % bis 40 % Bienenpollen und
- 60 % bis 80 % Ton.

6. Kombination nach einem der vorhergehenden Ansprüche, wobei sie eine Granulometrie kleiner als 500 µm, einen Feuchtigkeitsgehalt kleiner als 15 % und/oder eine Wasseraktivität (aw) kleiner als 0,75 aufweist.

7. Verfahren zur Herstellung einer Kombination nach einem der vorhergehenden Ansprüche, umfassend den Schritt des Mischens von Bienenpollen und Ton.

8. Pharmazeutische Zusammensetzung, enthaltend eine Kombination nach einem der Ansprüche 1 bis 6 und optional einen oder mehrere pharmazeutisch verträgliche(n) Träger.

9. Kombination nach einem der Ansprüche 1 bis 6 für die Verwendung in der Prävention und/oder der Behandlung von Osteoporose, von Herz-Kreislauf-Erkrankungen, von Krebserkrankungen, von Leberstörungen oder von metabolischen Störungen, von Kreislaufbeschwerden, von Symptomen der Menopause, von Störungen des Nervensystems, von Störungen des Magen-Darm-Trakts, von Okularstörungen, der Rekonvaleszenz, der Postchirurgie von Gelenkbeschwerden, von Urinbeschwerden, von Störungen der Prostata wie die benigne Prostatahyperplasie, die chronische Prostatitis, die Prostatodynie und der Prostatakrebs, den Ernährungsbeschwerden, dermatologischen Beschwerden, Asthenie und hydroelektrolytischen Ungleichgewichten.

10. Lebensmittelzusammensetzung, enthaltend eine Kombination nach einem der Ansprüche 1 bis 6.

11. Kapsel, enthaltend eine Kombination bestehend aus Ton und Bienenpollen nach einem der Ansprüche 1 bis 5.

12. Arzneimittel, als Träger enthaltend eine Kombination bestehend aus Ton und Bienenpollen nach einem der Ansprüche 1 bis 5.

13. Nicht-therapeutische Verwendung einer Zusammensetzung nach einem der Ansprüche 1 bis 5 bei der Behandlung von Erschöpfung.

## Claims

1. A combination consisting in beepollen and clay.

2. The combination according to claim 1, such that the clay is selected from clays of the kind: kaolinites, smectites, illites and chlorites or fibrous minerals.

3. The combination according to claim 1 or 2, such that the clay is montmorillonite.

4. The combination according to any of the preceding claims, consisting :
- from 4% to 90% of beepollen, and
- from 10% to 96% of clay,
the percentages being understood by weight.

5. The combination according to any of the main claims consisting :
- from 20% to 40% of beepollen and
- from 60% to 80% of clay.

6. The combination according to any of the preceding claims, such that it has a grain size of less than 500 µm, a humidity level of less than 15% and/or a water activity (wa) of less than 0.75.

7. A method for preparing a combination according to any of the preceding claims comprising the step for mixing beepollen and clay.

8. A pharmaceutical composition comprising a combination according to any of claims 1 to 6 and optionally one or several pharmaceutically acceptable excipients.

9. The combination according to any of claims 1 to 6 for use in preventing and/or treating osteoporosis, cardio-vascular diseases, cancers, liver and metabolism disorders, circulatory disorders, menopausal symptoms, disorders of the nervous system, disorders of the gastro-intestinal system, ocular disorders, convalescence, post-surgery articular disorders, urinary disorders, prostate disorders such as benign hypertrophia of the prostate, chronic prostatitis, prostatodynia and prostate cancer, nutritional disorders, dermatological disorders, asthenia and , hydro-electrolytic imbalances.

10. A food composition comprising a combination of any of claims 1 to 6.

11. A gelatin capsule containing a combination of clay and beepollen according to any of claims 1 to 6.

12. A drug comprising as a carrier, a combination consisting in clay and beepollen according to any of claims 1 to 6.

13. Non-therapeutical use of a composition according to any one of claims 1 to 5 for the treatment of fatigue.
